# EUROPÄISCHE PATENTSCHRIFT

(11) **EP 1 427 686 B1**
(45) Veröffentlichungstag und Bekanntmachung des Hinweises auf die Patenterteilung: **24.01.2007**
(21) Anmeldenummer: 02797551.5
(22) Anmeldetag: 28.08.2002
(51) Int. Cl.: C07C 6/04, C07C 11/06, C07C 6/10

(54) **VERFAHREN ZUR HERSTELLUNG VON PROPEN DURCH METATHESE VON C 4- BIS C 9-OLEFINEN**
METHOD FOR THE PRODUCTION OF PROPENE BY METATHESIS OF C 4- TO C 9- OLEFINS
PROCEDE DE PRODUCTION DE PROPENE PAR METATHESE D'OLEFINES C 4 A C 9

(30) Priorität: 04.09.2001 DE 10143160
(43) Veröffentlichungstag der Anmeldung: 16.06.2004
(73) Patentinhaber: BASF Aktiengesellschaft, 67056 Ludwigshafen (DE)
(72) Erfinder: RÖPER, Michael, 67157 Wachenheim (DE); STEPHAN, Jürgen, 68163 Mannheim (DE)
(86) Internationale Anmeldenummer: PCT/EP2002/009580
(87) Internationale Veröffentlichungsnummer: WO 2003/020669

(56) Entgegenhaltungen:
- EP-A- 0 832 867
- DE-A- 19 746 040
- US-B2- 6 271 430

## Beschreibung

Die vorliegende Erfindung betrifft ein Verfahren zur Herstellung von Propen durch mehrfaches Durchlaufen der nachfolgend genannten Schrittfolge, wobei man
I. in Schritt I eine Mischung M1 bestehend im wesentlichen aus
   - unverzweigten C₄- bis C₉ Monoolefinen (Komponenten 04-9),
   - 0 bis 15 mol-% Ethylen (Komponente E), bezogen auf die Komponente 04-9,
   - 0 bis 100 mol-% gesättigten Kohlenwasserstoffen (Komponenten KG), bezogen auf die Komponenten 04-9,
   - 0 bis 30 mol-%, bezogen auf die Komponenten 04-9, an Olefinen mit mehr als 9 Kohlenwasserstoffatomen (Komponenten 0>9)
   - 0 bis 1 mol-% bezogen auf die Komponenten 04-9, an Propen (Komponente O3)
   - 0 bis 10 mol-%, bezogen auf die Komponenten 04-9, an sonstigen Kohlenwasserstoffen (Komponenten KS)
   bei einer Temperatur von 20 bis 350°C mit einem Metathesekatalysator in Kontakt bringt, mit der Maßgabe, daß der molare Anteil der Summe der 1-Olefine und der Summe der 2-Olefine an der Summe der Komponenten 04-9 und 0>9 jeweils mindestens 0,5 % beträgt, sofern nicht die Mischung M1 gleichzeitig oder vorher einer Isomerisierung unterworfen wird, durch die der Anteil an 1- und 2-Olefin entsprechend eingestellt wird, und so eine Mischung M2 herstellt
II. in Schritt II aus der Mischung M2 eine Mischung M1 herstellt, indem man
   a) Propen und ggf. Komponenten 0>9 und Komponenten KS ganz oder teilweise entfernt und
   b) Komponenten 04-09, maximal 1 mol-% an Komponente E , bezogen auf die hinzugefügte Mengen an Komponenten 04-09, und ggf sonstige gesättigte oder olefinisch-ungesättigte Kohlenwasserstoffe, hinzufügt.

Es ist allgemein bekannt, daß mit Naphtha betriebene Steamcracker vor allem dazu genutzt werden, um ungesättigte Kohlenwasserstoffe bereitzustellen, die als Ausgangsprodukte für die Herstellung weiterer höher veredelter organischer Verbindungen dienen können. Besonders wertvolle Ausgangsprodukte sind Ethylen, Propylen, Butene und einen Phenylring enthaltende Kohlenwasserstoffe. Da einerseits das Produktespektrum des Steamcrackers, was die genannten Wertprodukte betrifft, nur in engen Grenzen beeinflußbar ist, andererseits der Bedarf an den einzelnen Wertprodukten z.T. jedoch sehr unterschiedlich ist, besteht ein besonderes Interesse daran, Verfahren bereitzustellen, einzelne dieser Wertprodukte, die lokal oder zeitlich bedingt in geringerem Ausmaß als andere benötigt werden, ineinander umzuwandeln, um so auf den jeweiligen Bedarf an den einzelnen Wertprodukten flexibel regieren zu können.

Ein häufig auftretendes Problem ist es, daß Olefine mit 4 und mehr Kohlenstoffatomen in ausreichendem Umfang zur Verfügung stehen, jedoch Propen und Ethylen besonders gefragt sind.

Verfahren zur Herstellung von Propen durch Metathesereaktionen aus anderen olefinischen Kohlenwasserstoffen sind z.B. aus folgenden Dokumenten bekannt.

Aus US 3785957 durch Umsetzung von 1- mit 2-Buten mit einem hohen Anteil an Ethylen an MoO₃ und CoO auf Al₂O₃.

Die EP-A-691318 betrifft ein Verfahren zur Herstellung von Butenen und Propenen aus Ethylen und Pentenen.

Aus der DE-A-19932060 ist eine Verfahren zur Herstellung von C5-/C6-Olefinen, wobei ebenfalls Propen erzeugt wird, bekannt. Dabei werden Butene einer Metathese unterworfen und aus der Reaktionsmischung Ethylen, Propen Pentene und Hexene abgetrennt. Lediglich das nicht umgesetzte Buten wird als Kreisstrom zurückgeführt.

Die DE-A-19813720 sowie die nicht-vorveröffentlichten DE-A-10013253, 10130958 und 10118634 betreffen Verfahren zur Umwandlung von C4- und höheren Olefinen zu Propen. Hierbei werden jedoch in verhältnismäßig großen Mengen Ethylen eingesetzt, welches bei der Metathese verbraucht wird.

Aufgabe der vorliegenden Erfindung war es deshalb, ein Verfahren bereitzustellen, mit dem man im wesentlichen ausschließlich ausgehend von olefinischen Kohlenwasserstoffen mit 4 und mehr Kohlenstoffatomen gezielt Propen herstellen kann.

EP-A-0 832 867, DE-A-197 46 040, US-B-6 271 4301 offenbaren Verfahren zur Herstellung von Propen.

Die erfindungsgemäß eingesetzte Mischung M1 besteht bevorzugt im wesentlichen aus
- unverzweigten C₄- bis C₉ Monoolefine (Komponente 04-9),
- 0,01 bis 15 mol-% Ethylen (Komponente E), bezogen auf die Komponente 04-9,
- 0 bis 100 mol-% gesättigten Kohlenwasserstoffen (Komponenten KG), bezogen auf die Komponente 04-9,
- 0 bis 30 mol-%, bezogen auf die Komponenten 04-9, an Olefinen mit mehr als 9 Kohlenwasserstoffatomen (Komponenten 0>9)
- 0 bis 1 mol-% bezogen auf die Komponente 04-9, an Propen (Komponente O3)
- 0 bis 10 mol-%, bezogen auf die Komponenten 04-9, an sonstigen Kohlenwasserstoffen (Komponenten KS)

Als Komponenten 04-9 kommen vor allem 1- und 2-Butene, n-Pentene n-Hexene, n-Octene und n-Nonene in Betracht.

Bei den Komponenten O>9 handelt es sich vor allem um unverzweigte Kohlenwassserstoffe mit einer olefinischen Doppelbindung wie Decene, Undecene oder Dodecene und Homologe.

Als gesättigte Kohlenwasserstoffe (Komponenten KG) kommen vor allem gesättigte Kohlenwasserstoffe wie Ethan, Propan, Butane, Isobutane, Neopentan, Isopentan, Methylcyclopropan in Betracht.
Als sonstige Kohlenwasserstoffe (Komponenten KS) kommen vor allem aromatische Kohlenwasserstoffe wie Benzol oder Styrol oder mehrfach ungesättigte Kohlenwsserstoffe wie 1,3-Butadien in Betracht.

Der Anteile an 1- und 2-Olefinen an der Summe der Komponenten 04-9 und 0>9 kann frei gewählt werden, wenn die Metathesereaktion unter Bedingungen durchgeführt wird, unter denen gleichzeitig eine statistische Isomerisierung Komponenten 04-9 stattfindet. Sofern diese Bedingung nicht erfüllt ist, beträgt der molare Anteil der Summe der 1-Olefine und der Summe der 2-Olefine an der Summe der Komponenten 04-9 und O>9 jeweils mindestens 0,5 %.

Als Metathesekatalysatoren, mit denen man die Mischung M1 für die gewünschte Reaktion in Kontakt bringt, wenn der definitionsgemäße Anteil der 1- und 2-Isomere vorliegt, eignen sich Katalysatoren, enthaltend eine Verbindung eines Metalls der Gruppen VIb oder VIIb des Priodensystems der Elemente. Vorzugsweise enthält der Metathesekatalysator ein Oxid eines Metalls der Gruppe VIb oder VIIb des Periodensystems. Insbesondere ist der Metathesekatalysator ausgewählt aus der Gruppe bestehend aus Re₂O₇, WO₃ und MoO₃.

Solche geeigneten Katalysatoren und deren Herstellung sind zB beschrieben in DE-A-10013253.

Die Umsetzung kann sowohl in der Flüssig- als auch in der Gasphase durchgeführt werden.

In flüssiger Phase wird die Metathese bevorzugt bei 0 bis 110°C und in der Gasphase bei 150 bis 350°C durchgeführt.

Der Druck beträgt im allgemeinen 10 bis 15 bar, sofern in der Flüssigphase und 1 bis 5 bar sofern in der Gasphase gearbeitet wird.

Üblicherweise sind Reaktionszeiten von 1 bis 5 h ausreichend.

Soll die Metathese unter isomerisierenden Bedingungen durchgeführt werden, was unabhängig vom Anteil von 1- und 2-Olefinen, bezogen auf die Komponenten 04-9 und 0>9 in der Mischung M1, generell bevorzugt ist, so stehen 2 Möglichkeiten zur Auswahl:
1. Man arbeitet bei Temperaturen von 110 bis 350°C und Drücken von 1 bis 60 bar, besonders bevorzugt bei etwa 150°C und etwa 5 bar mit Re₂O₇ auf Al₂O₃ als Katalysator.
2. Man setzt Katalysatorpackungen ein, die neben den vorgenannten Metathesekatalysatoren auch hiervon verschiedene Isomerisierungskatalysatoren enthalten. Die Isomerisierungskatalysatoren enthalten ein Metall aus den Gruppen Ia, IIa, IIIb, IVb, Vb oder VIII des Periodensystems der Elemente oder eine Verbindung davon. Vorzugsweise ist der Isomerisierungskatalysator ausgewählt aus der Gruppe bestehend aus RuO₂, MgO und K₂CO₃.
Die Katalysatoren sind generell auf den üblichen, dem Fachmann bekannten Materialien geträgert. Beispiele für geeignete Materialien umfassen SiO₂, gamma-Al₂O₃, MgO oder Mischungen dieser Materialien.

Die erfindungsgemäße Umsetzung kann diskontinuierlich oder kontinuierlich erfolgen, z.B. indem man einen Flüssig- oder Gasstrom, gebildet aus der Mischung M1, kontinuierlich in eine Reaktionszone leitet, dort mit dem Metathesekatalysator in Kontakt bringt und aus der Reaktionszone kontinuierlich einen Stoffstrom II entfernt.

Im allgemeinen wird in Schritt II aus der Mischung M2 wiederum kontinuierlich eine Mischung M1 hergestellt, indem man Stoffstrom II
a) Propen und ggf. Komponenten 0>9, KG und KS ganz oder teilweise enzieht (Schritt IIa) und
b) solche Mengen an Komponenten 04-09, maximal 1 mol-% an Komponente E und ggf sonstige gesättigte oder olefinisch-ungesättigte Kohlenwasserstoffe, bezogen auf die zugefügte Mengen an Komponenten 04-09, zusetzt(Schritt IIb).

Als die in Schritt IIa entfernten Komponenten sind nur solche Komponente zu verstehen, die aus dem System endgültig abgetrennt werden und somit nicht mehr zur Herstellung von Mischung M1 verwendet werden, jedoch nicht solche, die in einem Zwischenschritt oder Zwischenstufe von Schritt I oder II aus der Mischung M1 oder M2 oder daraus entfernten Bestandteilen zunächst abgetrennt werden, anschließend jedoch wieder zurückgeführt werden. Gleiches gilt sinngemäß für die in Schritt IIb hinzugefügten Komponenten, d.h. hierzu zählen nur solche, die dem System von außen hinzugefügt werden. Hierzu zählen folglich nicht solche Komponenten, die zunächst der Mischung M1 oder M2 oder einem daraus entfernten Bestandteil zunächst abgetrennt wurden und anschließend wieder zur Herstellung der Mischung 1 verwendet werden.

Eine Menge einer bestimmten Komponente gilt auch dann nicht als im Sinne von Schritt IIa als entfernt, wenn sie zwar aus dem System entfernt wird und nicht rückgeführt wird, jedoch die gleiche Menge der gleichen Komponente, die aus einer anderen Quelle stammt, der Mischung M1 oder M2 wieder hinzugefügt wird. Dies gilt auch entsprechend für die gemäß 2b hinzugefügten Komponenten.

Schritte I und II stellen die beiden Schritte einer Schrittfolge dar, die mehrfach wiederholt wird, was bevorzugt auf die Weise geschieht, daß es sich um einen kontinuierlich betriebenes Verfahren mit einem permanenten Stoffstrom handelt, der abwechselnd die Schritte I und II durchläuft.

Im allgemeinen wird in Schritt II so vorgegangen, daß die Masse der Summe an hinzugefügten Stoffmengen der Masse der Summe an entfernten Stoffmengen im wesentlichen entspricht.

Die Entfernung des Propens aus der Mischung M2 wird bevorzugt durchführt, indem man aus der Mischung M2 Propen und ggf. darin vorhandenes Ethylen von den höhersiedenden Komponenten abdestilliert und das Ethylen sowie den Destillationsrückstand zur Herstellung von Mischung M1 verwendet. Das so zurückgeführte Ethylen ist, wie vorstehend ausgeführt, kein Ethylen, das bei der Berechnung der gemäß Schritt IIb maximal zugelassenen hinzugefügten Ethylenmenge Berücksichtigung fände.

Die Entfernung von Komponenten 0>9 wird bevorzugt ebenfalls destillativ durchgeführt. Dies geschieht bevorzugt, um eine Anreicherung dieser Komponenten über den definitionsgemäßen Maximalgehalt in Mischung M1 zu verhindern.

Das Hinzufügen der Komponenten 04-9 in der Form bewirkt, daß man einer Mischung M2, aus der gemäß Schritt IIb Propen und ggf. Komponente O>9 entfernt wurde, eine Mischung M3 zugesetzt wird, bestehend aus Komponenten 04-09, maximal 1 mol-% an Komponente E, bezogen auf die zugefügte Mengen an Komponenten 04-09, und ggf sonstige gesättigte oder olefinisch-ungesättigte Kohlenwasserstoffe zusetzt.

Bevorzugt besteht die Mischung M3 im wesentlichen aus n-Butenen, n-Pentenen oder n-Hexenen oder Mischungen hiervon.

Es können als Mischungen M3 durch FCC-Cracken, o. verwandte Prozesse hergestellte Mischungen, weiterhin aus vorigen Metatheseprozessen isolierte n-Pentenschnitte oder Hexenschnitte eingesetzt werden.

Weiterhin kommen als Mischungen M3 in Betracht: Olefinschnitte aus thermischen Crackprozessen wie Steamcracking oder Raffinerieprozesse (Visbreaking, Flexicoking, delayed Coking). Für das thermische Cracken können dabei beliebige Edukte verwendet werden, bekannt sind beispielsweise Ethan, Propan, Butan, Raffinat II, Naphtha, Gasöl oder Vakuumgasöl.

Weiterhin können für das erfindungsgemässe Verfahren Mischungen M3 verwendet werden, die durch katalytisches Cracken erhalten werden (FCC = Fluidized Catalyst Cracking oder auch Hydrocrakking).

Nach angemessener Aufarbeitung sind alle diese Ströme als Mischungen M3 geeignet.

Die Mischung M3 wird bevorzugt bereitgestellt, indem man
- Naphtha oder eine sonstige Kohlenwasserstoffverbindung einem Steamcracking- oder *FCC* -Prozess (Fluid-Catalytic-Cracking-Przess) unterwirft und aus dem dabei gebildeteten Stoffstrom einen C₄-Kohlenwasserstofffraktion abzieht
- aus der C₄-Kohlenwasserstofffraktion einen im wesentlichen aus Isobuten, 1,-Buten, 2-Buten und Butanen bestehenden C₄-Kohlenwasserstoffstrom (Raffinat I) herstellt, indem man mittels Selektivhydrierung die Butadiene und Butine zu Butenen oder Butanen hydriert oder die Butadiene und Butine durch Extraktivdestillation entfernt
- aus dem Raffinat I den wesentlichen Anteil des Isobutens durch chemische, physikalisch-chemische oder physikalische Methoden abtrennt und auf diese Weise ein Raffinat II erhält,
- das Raffinat II durch Behandlung mit Adsorbermaterialien von Katalysatorgiften befreit und auf diese Weise Mischung M3 erhält.

Einzelheiten zu der Vorgehensweise bei diesen Schritten sind allgemein bekannt und können ebenfalls der DE-A-10013253 entnommen werden.

Das erfindungsgemäße Verfahren bietet den Vorteil, daß man ohne oder mit nur geringfügigem Verbrauch von extern zugeführtem Ethylen aus C4- und höheren olefinischen Kohlenwasserstoffen durch deren Metathese Propen herstellen kann. Dies gelingt besonders effektiv, wenn man vor oder gleichzeitig mit der Metathese die C₄- und höheren olefinischen Kohlenwasserstoffe einer Isomerisierung unterwirft, wobei 1- und 2-Olefine und Ethylen gebildet werden. Bei der isomerisierenden Metathese gebildetes Ethylen und 1-Olefine reagieren mit 2-Olefinen zu Propen.

Die bevorzugte Ausführungsform der Erfindung beruht also auf der vorteilhaften Kombination von 4 Reaktionen:
a) Selbstmetathese von zwei 1-Olefinen zu Ethylen und internem Olefin (z.B. 1-Buten + 1-Buten → Ethylen + 3-Hexen)
b) Ethenolyse von 2-Olefinen mit Ethylen zu Propylen und einem 1-Olefin (z.B. 2-Buten + Ethylen → Propen + Propen oder 2-Hexen + Ethylen → Propen + 1-Penten)
c) Kreuzmetathese von 1-Olefinen und 2-Olefinen (z.B. 1-Buten + 2-Buten → Propen + 2-Penten
d) statistischen Isomerisierung, um wieder einen gewissen Anteil an 1- und 2-Olefinen zu erzeugen (z.B. 1- oder 2-Hexen aus 3-Hexen)

## Patentansprüche

1. Verfahren zur Herstellung von Propen durch mehrfaches Durchlaufen der nachfolgend genannten Schrittfolge, wobei man
I. in Schritt I eine Mischung M1 bestehend im wesentlichen aus
- unverzweigten C₄- bis C₉ Monoolefinen (Komponenten 04-9),
- 0 bis 15 mol-% Ethylen (Komponente E), bezogen auf die Komponente 04-9,
- 0 bis 100 mol-% gesättigten Kohlenwasserstoffen (Komponenten KG), bezogen auf die Komponenten 04-9,
- 0 bis 30 mol-%, bezogen auf die Komponenten O4-9, an Olefinen mit mehr als 9 Kohlenwasserstoffatomen (Komponenten 0>9)
- 0 bis 1 mol-% bezogen auf die Komponenten 04-9, an Propen (Komponente O3)
- 0 bis 10 mol-%, bezogen auf die Komponenten 04-9, an sonstigen Kohlenwasserstoffen (Komponenten KS)
bei einer Temperatur von 20 bis 350°C mit einem Metathesekatalysator in Kontakt bringt, mit der Maßgabe, daß der molare Anteil der Summe der 1-Olefine und der Summe der 2-Olefine an der Summe der Komponenten 04-9 und O>9 jeweils mindestens 0,5 % beträgt, sofern nicht die Mischung M1 gleichzeitig oder vorher einer Isomerisierung unterworfen wird, durch die der Anteil an 1- und 2-Olefin entsprechend eingestellt wird, und so eine Mischung M2 herstellt
II. in Schritt II aus der Mischung M2 eine Mischung M1 herstellt, indem man
a) Propen und ggf. Komponenten 0>9 und Komponenten KS ganz oder teilweise entfernt und
b) Komponenten 04-09, maximal 1 mol-% an Komponente E , bezogen auf die hinzugefügte Mengen an Komponenten 04-09, und ggf. sonstige gesättigte oder olefinisch-ungesättigte Kohlenwasserstoffe hinzufügt.

2. Verfahren nach Anspruch 1, wobei man als Metathesekatalysatoren Verbindungen eines Metalls, das der VI.b., VII.b. oder VIII. Nebengruppe angehört, einsetzt.

3. Verfahren nach den Ansprüchen 1 oder 2, wobei man die geichzeitig mit der Metathese durchgeführte Isomerisierung in Schritt 1 bewirkt, indem man entweder
- als Metathesekatalysator Re₂O₇ auf Al₂O₃ verwendet und die Mischung M1 mit dem Katalysator bei Temperaturen von 110 bis 350°C und Drücken von 1 bis 60 bar in Kontakt bringt, oder
- die Mischung M1 mit Katalysatorpackungen in Kontakt bringt, die neben einem Metall, das der VI.b., VII.b. oder VIII. Nebengruppe angehört, auch übliche Isomerisierungskatalysatoren enthalten.

4. Verfahren nach den Ansprüchen 1 bis 3, wobei man in Schritt II die Komponente O>9 in solchen Mengen abtrennt, daß sich ihr Anteil in M1 in 2 aufeinanderfolgenden Schrittfolgen nicht erhöht.

5. Verfahren nach den Ansprüchen 1 bis 4, wobei man das Hinzufügen der Komponenten 04-9 in der Form bewirkt, daß man eine Mischung M3, bestehend im wesentlichen aus n-Butenen, n-Pentenen oder n-Hexenen oder Mischungen hiervon einsetzt.

6. Verfahren nach den Anspruch 5, wobei man die Mischung M3 herstellt, indem man
- Naphtha oder sonstige Kohlenwasserstoffverbindungen einem Steamcracking- oder FCC-Prozess unterwirft und aus dem dabei gebildeten Stoffstrom eine C₄-Kohlenwasserstofffraktion abzieht
- aus der C₄-Kohlenwasserstofffraktion einen im wesentlichen aus Isobuten, 1,-Buten, 2-Buten und Butanen bestehenden C₄-Kohlenwasserstoffstrom (Raffinat I) herstellt, indem man mittels Selektivhydrierung die Butadiene und Butine zu Butenen oder Butanen hydriert oder die Butadiene und Butine durch Extraktivdestillation entfernt
- aus dem Raffinat I den wesentlichen Anteil des Isobutens durch chemische, physikalisch-chemische oder physikalische Methoden abtrennt und auf diese Weise ein Raffinat II erhält
- das Raffinat II durch Behandlung mit Adsorbermaterialien von Katalysatorgiften befreit und auf diese Weise Mischung M3 erhält.

7. Verfahren nach den Ansprüchen 1 bis 5, wobei man die Abtrennung des Propens aus der Mischung M2 durchführt, indem man aus der Mischung M2 Propen und Ethylen von den höhersiedenden Komponenten andestilliert und das Ethylen sowie den Destillationsrückstand zur Herstellung von Mischung M1 verwendet.

8. Verfahren nach den Ansprüchen 1 bis 7, wobei die mehrfache Widerholung der Schrittfolge enthaltend die Schritte I und II in der Weise geschieht, daß es sich um einen kontinuierlich betriebenes Verfahren mit einem permanenten Stoffstrom handelt, der abwechselnd die Schritte I und II durchläuft.

## Claims

1. A process for preparing propene by carrying out the following sequence of steps a plurality of times, wherein
I. in step I, a mixture M1 consisting essentially of
- unbranched C₄-C₉-monoolefins (components 04-9),
- from 0 to 15 mol% of ethylene (component E), based on the components 04-9,
- from 0 to 100 mol% of saturated hydrocarbons (components HS), based on the components 04-9,
- from 0 to 30 mol%, based on the components 04-9, of olefins having more than 9 carbon atoms (components O>9)
- from 0 to 1 mol%, based on the components 04-9, of propene (component O3)
- from 0 to 10 mol%, based on the components 04-9, of other hydrocarbons (components HO)
is brought into contact with a metathesis catalyst at from 20 to 350°C, with the proviso that the mole fraction of all 1-olefins and of all 2-olefins as a proportion of the total components 04-9 and O>9 is in each case at least 0.5% if the mixture M1 is not subjected simultaneously or beforehand to an isomerization by means of which the proportion of 1- and 2-olefin is set accordingly, and a mixture M2 is thus prepared,
II. in step II, a mixture M1 is prepared from the mixture M2 by
a) removing propene and, if appropriate, components 0>9 and components HO either completely or partly and
b) adding components 04-09, a maximum of 1 mol% of component E, based on the amounts of components 04-09 added, and, if appropriate, other saturated or olefinically unsaturated hydrocarbons.

2. The process according to claim 1, wherein the metathesis catalyst used is a compound of a metal of transition group VI.b, VII.b or VIII.

3. The process according to claim 1 or 2, wherein the isomerization carried out simultaneously with the metathesis in step I is effected either by
- using Re₂O₇ on Al₂O₃ as metathesis catalyst and bringing the mixture M1 into contact with the catalyst at from 110 to 350°C and pressures of from 1 to 60 bar, or
- bringing the mixture M1 into contact with catalyst packing which comprises both a metal of transition group VI.b, VII.b or VIII and customary isomerization catalysts.

4. The process according to any of claims 1 to 3, wherein, in step II, the component O>9 is separated off in such amounts that its proportion in M1 does not increase in 2 successive sequences of the steps.

5. The process according to any of claims 1 to 4, wherein the addition of the components 04-9 is effected using a mixture M3 consisting essentially of n-butenes, n-pentenes or n-hexenes or mixtures thereof.

6. The process according to claim 5, wherein the mixture M3 is prepared by
- subjecting naphtha or other hydrocarbon compounds to a streamcracking or FCC process and taking off a C₄-hydrocarbon fraction from the stream formed,
- preparing a C₄-hydrocarbon stream consisting essentially of isobutene, 1-butene, 2-butene and butanes (raffinate I) from the C₄-hydrocarbon fraction by hydrogenating the butadienes and butynes to butenes or butanes by means of selective hydrogenation or removing the butadienes and butynes by extractive distillation,
- separating off the significant proportion of isobutene from the raffinate I by chemical, physicochemical or physical methods to give a raffinate II, and
- freeing the raffinate II of catalyst poisons by treatment with adsorber materials to give a mixture M3.

7. The process according to any of claims 1 to 5, wherein the propene is separated off from the mixture M2 by distilling off propene and ethylene from the mixture M2 to leave the higher-boiling components and using the ethylene and the distillation residue for preparing the mixture M1.

8. The process according to any of claims 1 to 7, wherein the multiple repetition of the sequence of steps I and II is carried out as a continuously operated process having a permanent stream of circulated material which is alternately subjected to the steps I and II.

## Revendications

1. Procédé de préparation de propène par passage à plusieurs reprises par la suite d'étapes citées dans la suite, dans lequel
I. dans l'étape I on met en contact un mélange M1 constitué essentiellement
- de monooléfines en C₄-C₉ non ramifiées (composants 04-9),
- de 0 à 15 % molaires d'éthylène (composant E) par rapport aux composants 04-9,
- de 0 à 100 % molaires d'hydrocarbures saturés (composants KG) par rapport aux composants 04-9,
- de 0 à 30 % molaires par rapport aux composants 04-9, d'oléfines comportant plus de 9 atomes de carbone (composant O>9),
- de 0 à 1 % molaire par rapport aux composants 04-9 de propène (composant O3),
- de 0 à 10 % molaires, par rapport aux composants 04-9, d'autres hydrocarbures (composants KS)
avec un catalyseur de métathèse, à une température de 20 à 350°C, à la condition que la fraction molaire de la somme des 1-oléfines et de la somme des 2-oléfines sur la somme des composants 04-9 et O>9 soit chaque fois d'au moins 0,5 %, dans la mesure où le mélange M1 n'est pas soumis simultanément ou auparavant à une isomérisation par laquelle la fraction de 1-oléfine et de 2-oléfine est ajustée de manière correspondante, et ainsi on prépare un mélange M2,
II. dans l'étape II, on prépare à partir du mélange M2 un mélange M1,
a) en éliminant totalement ou partiellement du propène et éventuellement des composants O>9 et des composants KS, et
b) en introduisant des composants 04-09, au maximum 1 mole % de composants E, par rapport aux quantités introduites de composants 04-09, et éventuellement d'autres hydrocarbures saturés ou oléfiniquement insaturés.

2. Procédé suivant la revendication 1, dans lequel, comme catalyseur de métathèse, on met en oeuvre des composés d'un métal qui appartient aux groupes secondaires VI.b., VII.b. ou VIII.

3. Procédé suivant les revendications 1 ou 2, dans lequel on produit l'isomérisation de l'étape 1 effectuée simultanément à la métathèse,
- en utilisant comme catalyseur de métathèse du Re₂O₇ sur du Al₂O₃ et en mettant en contact le mélange M1 avec le catalyseur à des températures de 110 à 350°C et à des pressions de 1 à 60 bars,
ou
- en mettant en contact le mélange M1 avec des garnissages de catalyseur qui contiennent, outre un métal qui appartient aux groupes secondaires VI.b., VII.b. ou VIII, également des catalyseurs d'isomérisation usuels.

4. Procédé suivant les revendications 1 à 3, dans lequel, dans l'étape II, on isole les composants O>9 en des quantités telles que leur fraction n'augmente pas dans M1 en deux suites d'étapes successives.

5. Procédé suivant les revendications 1 à 4, dans lequel on produit l'introduction des composants 04-9 dans la forme en mettant en oeuvre un mélange M3, constitué essentiellement de n-butènes, de n-pentènes ou de n-hexènes ou de leurs mélanges.

6. Procédé suivant la revendication 5, dans lequel on prépare le mélange M3
- en soumettant du naphte ou d'autres composés d'hydrocarbures à un processus de vapocraquage ou de FCC et en soutirant du courant de matière formé une fraction d'hydrocarbure en C₄,
- en préparant à partir de la fraction d'hydrocarbure en C₄ un courant d'hydrocarbures en C₄ constitué essentiellement d'isobutène, de 1-butène, de 2-butène et de butanes (produit de raffinage I), en hydrogénant au moyen d'une hydrogénation sélective les butadiènes et butynes en butènes et butanes ou en éliminant les butadiènes et butynes par une distillation par extraction,
- en isolant à partir du produit de raffinage I la fraction essentielle de l'isobutène par des procédés chimiques, physico-chimiques ou physiques et en obtenant de cette manière un produit de raffinage II,
- en libérant le produit de raffinage II des poisons de catalyseur par traitement avec des matières adsorbantes et en obtenant de cette manière un mélange M3.

7. Procédé suivant les revendications 1 à 5, dans lequel on effectue l'isolement du propène à partir du mélange M2 en séparant du mélange M2 du propène et de l'éthylène des composants à point d'ébullition supérieur par distillation et en utilisant l'éthylène ainsi que le résidu de distillation pour la préparation du mélange M1.

8. Procédé suivant les revendications 1 à 7, dans lequel la répétition à plusieurs reprises de la suite d'étapes contenant les étapes I et II s'effectue de manière qu'il s'agisse d'un procédé mis en oeuvre de manière continue avec un courant de matière permanent qui passe de manière alternante à travers les étapes I et II.
